Europäisches Patentamt

European Patent Office

Office européen des brevets

(11) Publication number: **0 092 363**
**B1**

## (12) EUROPEAN PATENT SPECIFICATION

(45) Date of publication of patent specification: **21.11.85**

(21) Application number: **83302042.3**

(22) Date of filing: **12.04.83**

(51) Int. Cl.⁴: **B 01 F 17/16, C 11 D 1/83,**
**C 11 D 1/75, C 11 D 1/22,**
**B 01 F 17/12, C 07 C 135/02**

(54) **Amine oxide formulations.**

(30) Priority: **13.04.82 GB 8210668**

(43) Date of publication of application:
**26.10.83 Bulletin 83/43**

(45) Publication of the grant of the patent:
**21.11.85 Bulletin 85/47**

(84) Designated Contracting States:
**AT BE CH DE FR GB IT LI LU NL SE**

(56) References cited:
**DE-A-2 853 171**
**DE-A-3 047 653**
**GB-A-1 087 414**
**GB-A-2 032 422**
**US-A-3 283 007**

(73) Proprietor: **Albright & Wilson Limited**
**Albright & Wilson House Hagley Road West**
**Oldbury Warley West Midlands, B68 ONN (GB)**

(72) Inventor: **Phillips, Brinley Morris**
**15 Greenlands Avenue**
**Whitehaven Cumbria (GB)**
Inventor: **Connor, David**
**28 Park Drive**
**Whitehaven Cumbria (GB)**

(74) Representative: **Savidge, Roger Gordon**
**Madgwick et al**
**c/o Albright & Wilson Limited 1 Knightsbridge**
**Green**
**London SW1X 7QD (GB)**

Courier Press, Leamington Spa, England.

## Description

The present invention relates to novel mixtures of surfactants, comprising amine oxides, which are available at high active concentrations.

Amine oxides are customarily prepared by reacting a trialkylamine having at least one long chain (e.g. $C_{10-20}$) alkyl group with hydrogen peroxide. Like many of the common industrial surfactants, amine oxides are soluble in water to provide mobile micellarsolutions up to a critical concentration which is usually about 30%. Above this critical concentration, the amine oxides form the immobile $M_1$ liquid crystal phase. Attempts to prepare amine oxides commercially at higher concentrations have been unsuccessful due to difficulty of adequately mixing the peroxide with the amine, and the intractable nature of the product.

For these reasons, amine oxides have always been manufactured using dilute hydrogen peroxide, and have been supplied and handled as dilute solutions. This is often highly inconvenient, and there has long been a demand for more highly concentrated amine oxides.

We have recently discovered that certain surfactants can be manufactured commercially as pourable fluids at substantially higher concentrations than the critical level at which an immobile $M_1$ phase occurs. This phenomenon is due to the formation, at a narrow range of very high concentrations, of a mobile "G" phase, which is a liquid crystal phase in which the surfactant molecules aggregate into lamellae of indefinite size separated by planes of water molecules.

Amine oxides have been obtained as a "G" phase in the laboratory, but the range of concentrations over which the "G" phase occurs is so narrow, e.g. between 69 and 73% by weight Active concentration in a typical instance, that it is impractical to manufacture them in this form on a plant scale. Slight variations of concentration, which are unavoidable on a commercial scale plant, cause rapid formation of immobile "$M_1$" gel, or of hydrated solid, which prevents continued operation of the equipment.

For the foregoing reasons amine oxides have not been commercially available as high active compositions, despite a substantial commercial demand for such products.

We have now discovered novel compositions comprising a high concentration of amine oxides which may readily be manufactured in the "G" phase in commercial plant and which are capable of satisfying to a substantial extent the demand for high active amine oxide compositions.

Our invention provides a surfactant composition consisting essentially of surfactant and water together, optionally, with a minor proportion e.g. from 0 to 10% by weight of non-surfactant electrolyte, said surfactant consisting essentially of an amine oxide having from one to two alky or alkenyl groups with from 10 to 20 carbon atoms and each other alkyl groups having from 1 to 4 carbon atoms, together with from 4 to 40% by weight of the total surfactant of an anionic surfactant, said composition having a total active concentration, being typically between 50 and 85% by weight, such that the composition is substantially in the "G" phase, i.e. above the $M_1$ phase/G phase boundary but below the G phase/ hydrated solid phase boundary.

All references herein to compositions comprising an amine oxide and an anionic surfactant include compositions comprising an amine oxide salt of an acid precursor of an anionic surfactant. References to total active concentrations include the total concentration of protonated amine oxide cation and surfactant anion of such salts, and references to percentages of amine oxide and of anionic surfactant, respectively include any protonated amine oxide cations and any surfactant anions of such salts.

The cationic and anionic components of the amine oxide salt may interact to diminish the surfactant properties of the mixture. This is not necessarily objectionable since high active compositions are commonly sold as intermediates in the manufacture of surfactant formulations. It may be convenient to adjust the pH of such formulations with base, which tends to restore the full surfactant properties.

The amine oxides are preferably $C_{10-20}$ alkyl or alkenyl dimethyl amine oxides wherein the $C_{10-20}$ alkyl or alkenyl groups may be a straight chain or branched chain primary or secondary group such as lauryl, cocoyl, cetyl, tallow, oleyl, linoleyl or octadecyl.

The anionic surfactant is preferably a water soluble sulphated or sulphonated surfactant salt, such as, preferably, an alkyl benzene sulphonate e.g. sodium dodecyl benzene sulphonate, or an alkyl sulphate, alkyl ether sulphate, paraffin sulphonate, olefin sulphonate, fatty acid sulphonate, fatty ester sulphonate, fatty acid alkanolamide sulphate, fatty acid alkanolamide ether sulphate, alkylphenol sulphate, or alkyl phenol ether sulphate. Alternatively the free acid precursor of any of the aforesaid surfactant salts may be added to form *in situ* the corresponding amine oxide salt, whose protonated amine oxide cation will then constitute at least part of the total amine oxide and whose surfactant anion will then constitute at least part of the total anionic surfactant. In each case the references in this paragraph to "alkyl" compounds is to be construed as including straight chain and branched chain, primary and secondary alkyl and alkenyl groups all having an average of from 10 to 20 carbon atoms. "Ether" is to be construed as including glyceryl ether and oxy-alkylene ethers comprising from 1 to 20 oxyethylene and/or oxypropylene groups. The cation may be, preferably, sodium or alternatively potassium, lithium, ammonium, a lower (e.g. $C_{1-6}$ preferably $C_{1-4}$) alkyl amine, or mono- di- or, less preferably, tri- ethanolamine.

Other anionic surfactants which may be used include soaps, alkyl- or alkylether- sulphosuccinates, alkyl or alkyl ether- sulphosuccinamates, acyl sarcosinates, acyl taurides, isethionates, alkyl ether carboxylates and anionic phosphate esters.

The non-surfactant electrolyte is preferably a water soluble inorganic salt, preferably of a strong base and strong acid such as sodium or potassium chloride, sodium or potassium sulphate, or sodium or potassium phosphate. Other non-surfactant electrolytes include the corresponding ammonium salts, and alkali metal or ammonium salts of lower molecular weight organic acids such as formates, glycollates, acetates, citrates and tartrates, and alkali metal or ammonium silicates and carbonates.

The proportion of anionic surfactant may be from 4 to 40% of the total weight of surfactant, preferably 7 to 30% e.g. 8 to 25%. The proportion of non-surfactant electrolyte is insufficient to suppress totally the formation of an immobile $M_1$ phase on dilution with water, e.g. up to 10% by weight. The concentration of non-surfactant electrolyte is preferably from 0 to 6% of the weight of the composition, e.g. 0 to 5%, especially 1 to 3%.

The composition is preferably substantially free from nonsurface-active organic material. The amount of any such material present should be insufficient to suppress totally the formation of insoluble $M_1$ phase on dilution with water. Surfactants often contain small amounts of non-surfactant organic material as manufacturing impurities but we prefer not to add such materials to compositions of our invention. In particular we prefer not to add solvents such as ethanol or other lower alcohols, or viscosity modifiers such as urea, which have in the past been included in surfactant compositions to increase the upper concentration of the micellar solution/$M_1$ phase boundary, and sometimes to suppress the $M_1$ phase altogether. Deliberate addition of such non-surfactant organic material increases the cost of the product, without contributing to surface activity and may adversely affect other properties of the composition or provide incompatible with the end user formulations.

The active concentration of the composition is such that the composition as a whole is present as a pourable, and at least predominantly "G" phase product. This will typically occur over a range of concentrations above 50% total active but usually below 80%. Depending upon the formulation, this range usually extends over from $\pm 3$ to $\pm 10\%$ from the minimum value in the viscosity/concentration curve corresponding to the "G" phase, which minimum typically occurs at a total active concentration between 53 and 75% depending upon the formulation.

The concentration range over which any given composition of the invention occurs in the "G" phase may be identified very rapidly and easily, using standard laboratory equipment by making a test composition having an active concentration of say 75% and placing a sample on a slide on the block of a heated stage microscope. Examination between crossed polarisers will reveal in which phase the sample is present. The various phases each have a characteristic appearance which is easily identified by comparison for example with the photographs of typical liquid crystal phases in the classic paper by Rosevear, JAOCS Vol. 31p 628 (1954) or in J. Colloid and Interfacial Science Vol. 30 No. 4 page 500.

If the mixture is in an $M_1$ phase, water may be allowed to evaporate from the edges of the sample under the cover disk and any phase changes observed. If any $M_2$ phase or hydrated solid is present water may be added around the edge of the cover disks and allowed to diffuse into the composition. If no "G" phase is located in this way samples may be heated progressively on the block and the operation repeated.

Compositions according to our invention may be prepared by reacting an appropriate tertiary amine with hydrogen peroxide in the presence of the anionnic surfactant, any non-surfactant electrolyte, and sufficient water to maintain the product in the "G" phase.

Alternatively, the amine may be reacted with a sulphonic or sulphuric acid surfactant precursor forming the amine salt. The latter is then reacted with hydrogen peroxide to form the amine oxide salt. If desired the pH of such substantially neutral, products may be subsequently raised by the addition of base to provide compositions equivalent to those obtained by peroxidising mixture of the amine with the anionic surfactant.

The invention will be illustrated by the following examples in which the alkyl dimethylamine was an n alkyl dimethyl amine, wherein the n alkyl group had the following specification:

| | |
|---|---|
| $C_{10}$ | $3\pm 1\%$ |
| $C_{12}$ | $67\pm 4\%$ |
| $C_{14}$ | $25\pm 3\%$ |
| $C_{16}$ | 8% max |

Example I

A stirred water jacketed reactor was charged as follows:

Dodecyl benzene sulphonic acid (DDBS)—(95%) 133.7g

$C_{12/14}$ alkyl dimethylamine—(98%) 687.9g (3.05 mol)

EDTA—2.0g.

The temperature was raised to 65°C and 210.7g of 50% hydrogen peroxide (3.10 mol) was added over a period of 4 hour maintaining the temperature at 70—80°C. The reaction mixture was then stirred at 65°C for 40 hours. The product has the following anaylsis:

| | |
|---|---|
| Amine oxide | 68.1% |
| DDBS | 13.5% |
| Unreacted amine | 0.3% |

The reaction product was then successively diluted with water after the addition of 5% by weight sodium chloride on the combined weight of DDBS and amine oxide, and the viscosity measured at 25°C.

| % Amine Oxide | Viscosity (Shear rate 8.51 s⁻¹) |
|---|---|
| 60% | Viscous paste |
| 58% | 8500 mpas |
| 54% | 3750 mpas* |
| 50% | 2800 mpas |
| 48% | 2900 mpas |
| 46% | Gel |

* Mobile G phase

Example II

Amine oxide, in the presence of DDBS, was prepared as described in Example I, except that the weight of DDBS was reduced to 62.3g and 37g water was added to the initial reaction mixture. The product had the following analysis.

| Amine oxide | 71% |
|---|---|
| DDBS | 6.4% |
| Unreacted amine | 0.5% |

The reaction product was then successively diluted with water after the addition of 2% by weight sodium chloride on the combined weight of DDBS and amine oxide, and the viscosity measured under the conditions described in Example I.

| % Amine Oxide | Viscosity |
|---|---|
| 66% | Paste |
| 64% | 3750 mpas* |
| 62% | 3500 mpas |
| 60% | 3500 mpas |
| 58% | 3700 mpas |
| 56% | 4750 mpas |
| 54% | 6500 mpas |
| 52% | Gel |

* Mobile G phase

Example III (Comparative)

A sample of concentrated amine oxide was prepared as described in Example I in the absence of co-surfactant. The material was successively diluted with water and the viscosity measured under the conditions described in Example I.

| % Amine Oxide | Viscosity |
|---|---|
| 75% | Paste |
| 74% | 8600 mpas |
| 72% | 5800 mpas* |
| 70% | 5800 mpas* |
| 69% | Gel |

* Mobile G phase

In this comparative example the amine oxide, alone, was pourable over a much narrower range than in the examples of this invention.

Example IV

The following blends were prepared using the technique described in Example I, and are compositions which are illustrative of the invention.

| % DDBS | 9.9 | 10.3 | 10.7 | 5.4 |
|---|---|---|---|---|
| % NaCl | 3.0 | — | 1.3 | 1.3 |
| % Amine Oxide | 50.0 | 52 | 54 | 50 |
| Viscosity @ 25°C (shear rate 8.51 s⁻¹) | 2800* | 3000* | 3000* | 3500* |

* Mobile G phase

**Claims**

1. A composition consisting essentially of surfactant and water, said surfactant consisting essentially of an amine oxide having from one to two alkyl or alkenyl groups with from 10 to 20 carbon atoms and each other alkyl group having from 1 to 4 carbon atoms, together with from 4 to 40%, by weight of the total surfactant, of an anionic surfactant, said composition having a total active concentration above that corresponding to the $M_1/G$ phase boundary and below that corresponding to the G phase/hydrated solid phase boundary.

2. A composition according to claim 1 containing a minor proportion of non-surfactant electrolyte.

3. A composition according to claim 2 contains up to 10% by weight of non-surfactant electrolyte.

4. A composition according to claim 3 wherein the non-surfactant electrolyte is a sodium, potassium or ammonium salt of a strong mineral acid or a lower carboxylic or hydroxy carboxylic acid.

5. A composition according to any foregoing claim wherein the anionic surfactant is an alkyl benzene sulphonate.

6. A composition according to any foregoing claim comprising an amine oxide salt of the sulphuric or sulphonic acid precursor of an anionic surfactant.

7. A method for the preparation of a composition according to any foregoing claim which comprises adding aqueous hydrogen peroxide to a mixture consisting essentially of trialkyl amine having from one to two alkyl or alkenyl groups with from 10 to 20 carbon atoms, each other alkyl group having from 1 to 4 carbon atoms, and from 4 to 40%, by weight of the total surfactant, of an anionic surfactant and adjusting the water content to maintain the mixture in the G phase.

## Patentansprüche

1. Zubereitung, bestehend hauptsächlich aus oberflächenaktiven Mitteln und Wasser, wobei das oberflächenaktive Mittel hauptsächlich aus einem Aminoxid mit 1 bis 2 Alkyl- oder Alkenylgruppen mit 10 bis 20 Kohlenstoffatomen und jeder weiteren Alkylgruppe mit 1 bis 4 Kohlenstoffatomen besteht zusammen mit 4 bis 40 Gew.-%, bezogen auf die Gesamtmenge ober-flächenaktives, Mittel eines anionischen ober-flächenaktiven Mittels, wobei die Zubereitung eine Gesamtkonzentration an aktiven Bestandteilen oberhalb derjenigen hat, die der Grenze der $M_1$/G-Phase entspricht, und unter derjenigen, die der Grenze G-Phase/hydratisierte feste Phase entspricht.

2. Zubereitung nach Anspruch 1, enthaltend eine kleinere Menge nicht-oberflächenaktiver Elektrolyt.

3. Zubereitung nach Anspruch 2, die bis zu 10 Gew.-% nicht-oberflächenaktiver Elektrolyt enthält.

4. Zubereitung nach Anspruch 3, wobei der nicht-oberflächenaktive Elektrolyt ein Natrium-, Kalium- oder Ammoniumsalz einer starken Mineralsäure oder einer niederen Carbonsäure oder Hydroxycarbonsäure ist.

5. Zubereitung nach den vorhergehenden Ansprüchen, wobei das anionische oberflächenaktive Mittel ein Alkyl-benzolsulfonat ist.

6. Zubereitung nach den vorhergehenden Ansprüche, enthaltend ein Aminooxidsalz der Vorläufers des anionischen oberflächenaktiven Mittels in Form der Schwefelsäure oder Sulfonsäure.

7. Verfahren zur Herstellung einer Zubereitung nach den vorhergehenden Ansprüchen, dadurch gekennzeichnet, daß wässriger Wasserstoffperoxid einem Gemisch zugegeben wird, das im wesentlichen aus Trialkylamin mit 1 bis 2 Alkyl- oder Alkenylgruppen mit 10 bis 20 Kohlenstoffatomen und jede weitere Alkylgruppe mit 1 bis 4 Kohlenstoffatomen und 4 bis 40 Gew.-%, bezogen auf die Gesamtmenge oberflächenaktive Mittel, eines anionischen oberflächenaktiven Mittels besteht und den Wassergehalt so einstellt, daß das Gemisch in der G-Phase gehalten wird.

## Revendications

1. Composition constituée essentiellement par un tensioactif et de l'eau, ledit tensioactif étant essentiellement composé d'un oxyde d'amine comportant 1 à 2 groupes alkyles ou alcényles avec 10 à 20 atomes de carbone et chaque autre groupe alkyle ayant 1 à 4 atomes de carbone, conjointement avec 4 à 40% en poids, par rapport au tensioactif total, d'une tensioactif anionique, ladite composition ayant une concentration totale an matière active supérieure à celle correspondant à la séparation phase $M_1$/phase G et inférieure à celle correspondant à la séparation phase G/phase solide hydraté.

2. Composition selon la revendication 1, contenant une faible proportion d'électrolyte non tensioactif.

3. Composition selon la revendication 2 contenant jusqu'à 10% en poids d'électrolyte non tensioactif.

4. Composition selon la revendication 3 dans laquelle l'électrolyte non tensioactif est un sel de sodium, de potassium ou d'ammonium d'un acide minéral fort ou d'un acide carboxylique ou· hydroxycarboxylique inférieur.

5. Composition selon l'une quelconque des revendications précédentes, dans laquelle le tensioactif anionique est une alkylbenzène-sulfonate.

6. Composition selon l'une quelconque des revendications précédentes, comprenant un sel d'oxyde d'amine du précurseur acide sulfurique ou sulfonique d'un tensioactif anionique.

7. Procédé pour la préparation d'une composition selon l'une quelconque des revendications précédentes, qui comprend: ajouter du peroxyde d'hydrogène aqueux à un mélange constitué essentiellement par un trialkylamine possédant un à deux groupes alkyles ou alcényles avec 10 à 20 atomes de carbone, chaque autre groupe alkyle ayant de 1 à 4 atomes de carbone et par 4 à 40% en poids de tensioactic total, d'un tensioactif anionique et ajuster la teneur en eau en vue de maintenir le mélange dans la phase G.